# EUROPEAN PATENT APPLICATION

(11) **EP 4 592 288 A1**
(43) Date of publication of application: **30.07.2025**
(21) Application number: 23867540.9
(22) Date of filing: 20.09.2023
(51) Int. Cl.: C07D 471/04, A61K 31/437, A61P 25/18

(54) **CRYSTAL FORM OF EMRACLIDINE, PREPARATION METHOD THEREFOR, AND USE THEREOF**

(30) Priority: 23.09.2022 CN 202211170599; 23.09.2022 CN 202211165395; 16.12.2022 CN 202211637732; 16.12.2022 CN 202211626881; 16.12.2022 CN 202211626754
(71) Applicant: Cerevel Therapeutics, LLC, Boston, MA 02116 (US)
(72) Inventor: QIAN, Jiale, Suzhou, Jiangsu 215123 (CN); HUANG, Chunxiang, Suzhou, Jiangsu 215123 (CN); MENG, Liping, Suzhou, Jiangsu 215123 (CN)
(74) Representative: J A Kemp LLP
(86) International application number: PCT/CN2023/119900
(87) International publication number: WO 2024/061253

(57) **Abstract**

The present invention relates to a crystal form of Emraclidine (hereinafter referred to as "compound I"), a preparation method therefor, a pharmaceutical composition containing the crystal form, and use of the crystal form in the preparation of an M4 receptor modulator medicament and a medicament for treating schizophrenia.

## Description

### TECHNICAL FIELD

The present invention relates to the field of crystal chemistry. In particular, the invention relates to the crystal form of Emraclidine, a preparation method and a use thereof.

### BACKGROUND ART

Schizophrenia is a mental health disorder which is serious, complex, and debilitating, characterized by a series of symptoms, including delusion, hallucinations, speech or behavioral disturbances, bradyphrasia, and affective blunting.

Emraclidine (CVL-231), developed by Cerevel Therapeutics, is a positive allosteric modulator (PAM) which selectively targets muscarinic acetylcholine 4 (M4) receptors for treating schizophrenia. Emraclidine shows, via a Phase-Ib clinical result thereof, clinically significant antipsychotic activities compared to placebos.

Emraclidine has a chemical name, 1-(2, 4-dimethyl-5,7-dihydro-6H-pyrrolo[3,4-b]pyridin-6-yl)-2-{ 1-[2-(trifluoromethyl)pyridin-4-yl]azetidin-3-yl} ethanone (hereinafter referred to as "compound I"), and the compound is disclosed in WO 2018002760 A1. Compound I has the following formula:

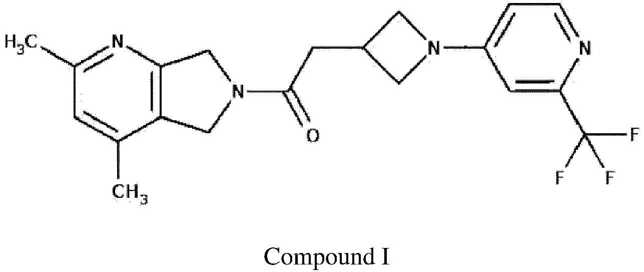

It is well known in the art that in small molecule drug development, drug polymorphism is a common phenomenon in drug development and research, and is an important factor affecting the quality of a drug. A crystal is a solid in which a crystal lattice is formed by three-dimensional ordered arrangement of compound molecules in a microstructure. Polymorphism refers to the phenomenon that a compound has multiple crystal forms. A compound may exist in one or more crystal forms, but the existence and properties of the crystal forms cannot be specifically expected. Drug substances with different crystal forms have different physicochemical properties, including chemical stability, thermal stability, solubility, hygroscopicity and/or a particle size, which may lead to different dissolution and absorption of a drug in vivo, thereby affecting the clinical efficacy of the drug to some extent. In addition, drug substances with different crystal forms have different manufacturability, including a yield, and purification, filtration, drying, and grinding properties, and the stability under pressure during tableting may affect processing of drug substances during production. Thus, the polymorphism is important for the research and quality control of a drug. Partly because of these reasons, there is a need to find a solid form of compound I.

The inventor of the present application unexpectedly found that the crystal of compound I provided by the present invention has good physicochemical stability, good stability under mechanical force, and low hygroscopicity, and is significant for developing a drug containing compound I.

### SUMMARY OF THE INVENTION

The present invention provides a crystal of compound I, a preparation method therefor, a pharmaceutical composition comprising the crystal, and a use thereof.

According to an object of the present invention, the present invention provides crystal form CSI of Compound I (hereinafter referred to as "crystal form CSI").

In one aspect, an X-ray powder diffraction pattern of the crystal form CSI comprises a characteristic peak at a diffraction angle 2θ value of 1, or 2, or 3 of 11.9°±0.2°, 5.9°±0.2°, and 9.5°±0.2° using Cu-Kα radiation; and preferably, the X-ray powder diffraction pattern of the crystal form CSI comprises characteristic peaks at diffraction angle 2θ values of 11.9°±0.2°, 5.9°±0.2°, and 9.5°±0.2°.

Further, the X-ray powder diffraction pattern of the crystal form CSI comprises a characteristic peak at a diffraction angle 2θ value of 1, or 2, or 3 of 10.7°±0.2°, 14.1±0.2°, and 22.2°±0.2° using Cu-Kα radiation; and preferably, the X-ray powder diffraction pattern of the crystal form CSI comprises characteristic peaks at diffraction angle 2θ values of 10.7°±0.2°, 14.1°±0.2°, and 22.2°±0.2°.

Further, the X-ray powder diffraction pattern of the crystal form CSI comprises a characteristic peak at a diffraction angle 2θ value of 1, or 2, or 3 of 10.4°±0.2°, 16.3°±0.2°, and 19.1°±0.2° using Cu-Kα radiation; and preferably, the X-ray powder diffraction pattern of the crystal form CSI comprises characteristic peaks at diffraction angle 2θ values of 10.4°±0.2°, 16.3°±0.2°, and 19.1°±0.2°.

In another aspect, the X-ray powder diffraction pattern of the crystal form CSI comprises a characteristic peak at a diffraction angle 2θ value of any 1, or 2, or 3, or 4, or 5, or 6, or 7, or 8, or 9, or 10, or 11, or 12, or 13 of 11.9±0.2°, 5.9°±0.2°, 9.5°±0.2°, 10.7°±0.2°, 14.1°±0.2°, 22.2°±0.2°, 10.4°±0.2°, 16.3°±0.2°, 19.1°±0.2°, 27.1°±0.2°, 21.0°±0.2°, 26.7°±0.2°, and 21.5°±0.2° using Cu-Kα radiation.

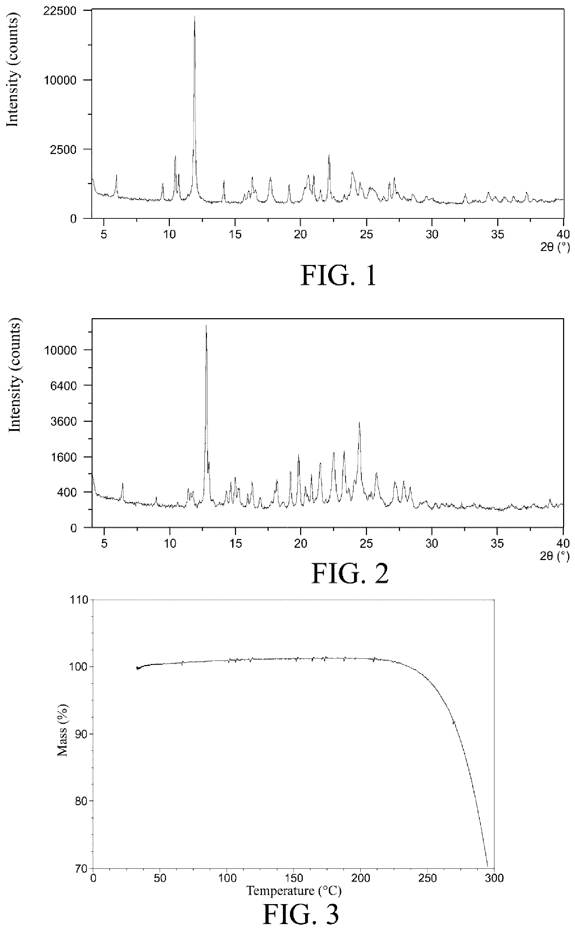

Without limitation, the X-ray powder diffraction pattern of the crystal form CSI is substantially as shown in FIG. 1 using Cu-Kα radiation.

According to an object of the present invention, the present invention also provides a method for preparing the crystal form CSI, the method comprising: dissolving Compound I in a ketone solvent to obtain the crystal form CSI via volatilization.

Further, the volatilization temperature is preferably -20°C-50°C.

According to an object of the present invention, the present invention provides a crystal form of Compound I, which is, without limitation, a crystal form CSII (hereinafter referred to as "crystal form CSII").

In one aspect, an X-ray powder diffraction pattern of the crystal form CSII comprises a characteristic peak at a diffraction angle 2θ value of 1, or 2, or 3 of 12.8°±0.2°, 19.8°±0.2°, and 24.4°±0.2° using Cu-Kα radiation; and preferably, the X-ray powder diffraction pattern of the crystal form CSII comprises characteristic peaks at diffraction angle 2θ values of 12.8°±0.2°, 19.8°±0.2°, and 24.4°±0.2°.

Further, the X-ray powder diffraction pattern of the crystal form CSII comprises a characteristic peak at a diffraction angle 2θ value of 1, or 2, or 3 of 21.5°±0.2°, 22.5°±0.2°, and 23.3°±0.2° using Cu-Kα radiation; and preferably, the X-ray powder diffraction pattern of the crystal form CSII comprises characteristic peaks at diffraction angle 2θ values of 21.5°±0.2°, 22.5°±0.2°, and 23.3°±0.2°.

Further, the X-ray powder diffraction pattern of the crystal form CSII comprises a characteristic peak at a diffraction angle 2θ value of 1, or 2, or 3 of 19.2°±0.2°, 20.8°±0.2°, and 25.8°±0.2° using Cu-Kα radiation; and preferably, the X-ray powder diffraction pattern of the crystal form CSII comprises characteristic peaks at diffraction angle 2θ values of 19.2°±0.2°, 20.8°±0.2°, and 25.8°±0.2°.

In another aspect, the X-ray powder diffraction pattern of the crystal form CSII comprises a characteristic peak at a diffraction angle 2θ value of any 1, or 2, or 3, or 4, or 5, or 6, or 7, or 8, or 9, or 10, or 11, or 12, or 13 of 12.8°±0.2°, 19.8°±0.2°, 24.4°±0.2°, 21.5°±0.2°, 22.5°±0.2°, 23.3°±0.2°, 19.2°±0.2°, 20.8°±0.2°, 25.8°±0.2°, 6.4°±0.2°, 9.0°±0.2°, 14.6°±0.2°, and 16.3°±0.2° using Cu-Kα radiation.

Without limitation, the X-ray powder diffraction pattern of the crystal form CSII is substantially as shown in FIG. 2 using Cu-Kα radiation.

Without limitation, a thermo gravimetric analysis thermogram of the crystal form CSII is substantially as shown in FIG. 3 with substantially no weight loss when heated from room temperature to 180°C.

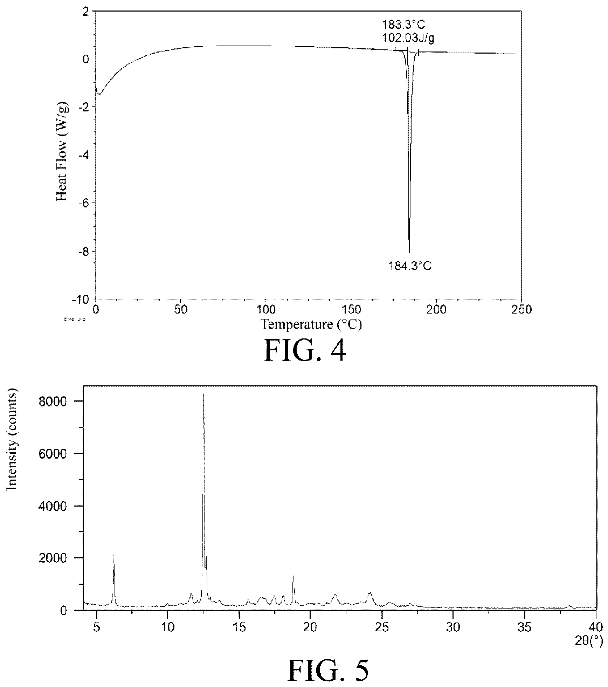

Without limitation, a differential scanning calorimetry thermogram of the crystal form CSII is substantially as shown in FIG. 4, with an endothermic peak beginning at about 183°C, which is a melting endothermic peak.

Without limitation, the crystal form CSII is an anhydrate.

According to an object of the present invention, the present invention also provides a method for preparing the crystal form CSII, the method comprising: placing Compound I in a mixed system of methyl t-butyl ether and alcohol to obtain the crystal form CSII by stirring.

Further, the alcohol is preferably methanol; and the stirring temperature is preferably 0°C-50°C, and the stirring time is preferably 1-10 day.

According to an object of the present invention, the present invention provides a crystal form of compound I, which is, without limitation, a crystal form CSIII (hereinafter referred to as "crystal form CSIII").

In one aspect, an X-ray powder diffraction pattern of the crystal form CSIII comprises a characteristic peak at a diffraction angle 2θ value of 1, or 2, or 3 of 12.5°±0.2°, 6.2°±0.2°, 18.8°±0.2°, and 17.5°±0.2° using Cu-Kα radiation; and preferably, the X-ray powder diffraction pattern of the crystal form CSIII comprises characteristic peaks at diffraction angle 2θ values of 12.5°±0.2°, 6.2°±0.2°, 18.8°±0.2°, and 17.5°±0.2°.

Further, the X-ray powder diffraction pattern of the crystal form CSIII comprises a characteristic peak at a diffraction angle 2θ value of 1 or 2 of 18.1+0.2°, and 21.7°±0.2° using Cu-Kα radiation; and preferably, the X-ray powder diffraction pattern of the crystal form CSIII comprises characteristic peaks at diffraction angle 2θ values of 18.1°±0.2°, and 21.7°±0.2°.

In another aspect, the X-ray powder diffraction pattern of the crystal form CSIII comprises a characteristic peak at a diffraction angle 2θ value of any 1, or 2, or 3, or 4, or 5, or 6, or 7, or 8 of 12.5°±0.2°, 6.2°±0.2°, 18.8°± 0.2° 17.5°±0.2°, 18.1°±0.2°, 21.7°±0.2°, 11.6°±0.2°, and 24.2±0.2° using Cu-Kα radiation.

Without limitation, the X-ray powder diffraction pattern of the crystal form CSIII is substantially as shown in FIG. 5 using Cu-Kα radiation.

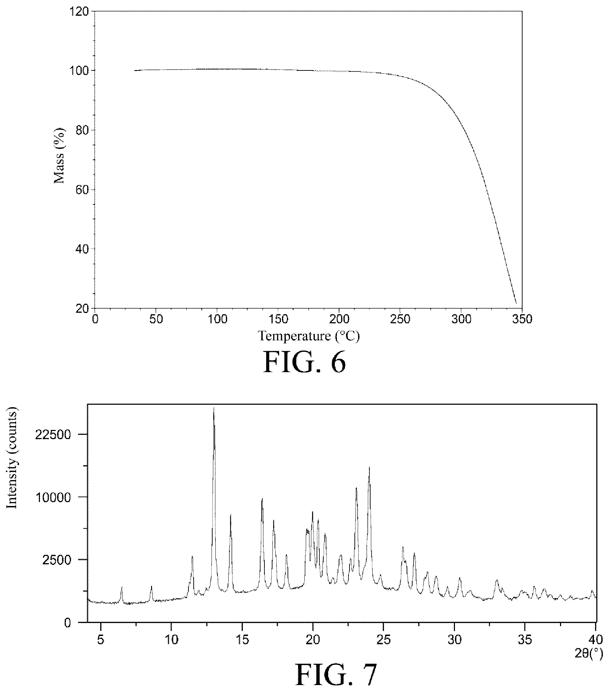

Without limitation, a thermo gravimetric analysis thermogram of the crystal form CSIII is substantially as shown in FIG. 6 with substantially no weight loss when heated from room temperature to 170°C.

Without limitation, the crystal form CSIII is an anhydrate.

According to an object of the present invention, the present invention also provides a method for preparing the crystal form CSIII, the method comprising: dissolving Compound I in a mixed solvent of an ether and an alkane to obtain the crystal form CSIII via volatilization.

Further, the ether is preferably tetrahydrofuran; the alkane is preferably n-heptane; and the volatilization temperature is preferably 40-60°C.

According to an object of the present invention, the present invention provides a crystal form of Compound I, which is, without limitation, a crystal form CSIV (hereinafter referred to as "crystal form CSIV").

In one aspect, an X-ray powder diffraction pattern of the crystal form CSIV comprises a characteristic peak at a diffraction angle 2θ value of 1, or 2, or 3, or 4 of 13.0°±0.2°, 16.4°±0.2°, 23.1°±0.2°, and 22.0°±0.2° using Cu-Kα radiation; and preferably, the X-ray powder diffraction pattern of the crystal form CSIV comprises characteristic peaks at diffraction angle 2θ values of 13.0°±0.2°, 16.4°±0.2°, 23.1°±0.2°, and 22.0°±0.2°.

Further, the X-ray powder diffraction pattern of the crystal form CSIV comprises a characteristic peak at a diffraction angle 2θ value of 1, or 2, or 3 of 24.0°±0.2°, 14.2°±0.2°, and 17.2°±0.2° using Cu-Kα radiation; and preferably, the X-ray powder diffraction pattern of the crystal form CSIV comprises characteristic peaks at diffraction angle 2θ values of 24.0°±0.2°, 14.2°±0.2°, and 17.2°±0.2°.

Further, the X-ray powder diffraction pattern of the crystal form CSIV shows a characteristic peak at a diffraction angle 2θ value of 1, or 2, or 3 of 18.1°±0.2°, 20.8±0.2°, and 27.2°±0.2° using Cu-Kα radiation; and preferably, the X-ray powder diffraction pattern of the crystal form CSIV comprises characteristic peaks at diffraction angle 2θ values of 18.1°±0.2°, 20.8°±0.2°, and 27.2°±0.2°.

In another aspect, the X-ray powder diffraction pattern of the crystal form CSIV comprises a characteristic peak at a diffraction angle 2θ value of any 1, or 2, or 3, or 4, or 5, or 6, or 7, or 8, or 9, or 10 of 13.0 °±0.2°, 16.4°±0.2°, 23.1°±0.2°, 22.0°±0.2°, 24.0°±0.2°, 14.2°±0.2°, 17.2°±0.2°, 18.1°±0.2°, 20.8°±0.2°, 27.2°±0.2°, 6.5°±0.2°, 8.6°±0.2°, 11.5, 20.0°±0.2°, 20.4°±0.2°, and 26.4°±0.2°.

Without limitation, the X-ray powder diffraction pattern of the crystal form CSIV is substantially as shown in FIG. 7 using Cu-Kα radiation.

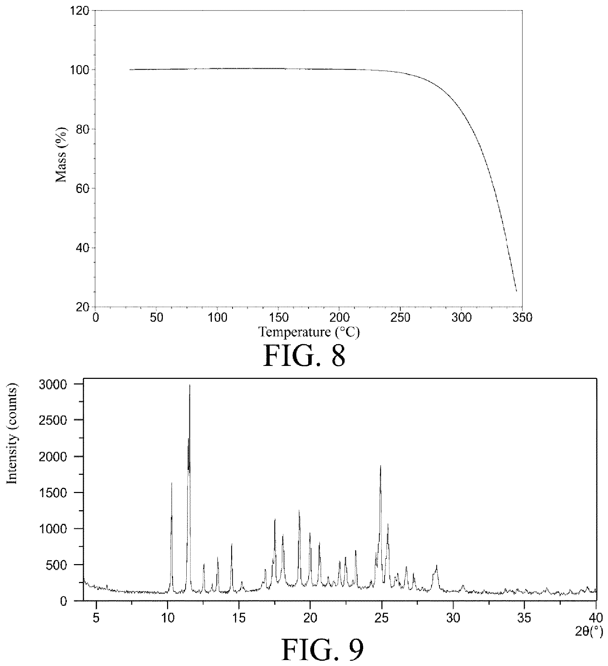

Without limitation, a thermo gravimetric analysis thermogram of the crystal form CSIV is substantially as shown in FIG. 8 with substantially no weight loss when heated from room temperature to 180°C.

Without limitation, the crystal form CSIV is an anhydrate.

According to an object of the present invention, the present invention also provides a method for preparing the crystal form CSIV, the method comprising: heating Compound I to 185°C-190°C to obtain the crystal form CSIV.

According to an object of the present invention, the present invention provides a crystal form of Compound I, which is, without limitation, a crystal form CSV (hereinafter referred to as "crystal form CSV").

In one aspect, an X-ray powder diffraction pattern of the crystal form CSV comprises a characteristic peak at a diffraction angle 2θ value of 1, or 2, or 3, or 4 of 10.3°±0.2°, 19.2°±0.2°, 13.5°±0.2°, and 14.5°±0.2° using Cu-Kα radiation; and preferably, the X-ray powder diffraction pattern of the crystal form CSV comprises characteristic peaks at diffraction angle 2θ values of 10.3°±0.2°, 19.2°±0.2°, 13.5°±0.2°, and 14.5°±0.2°.

Further, the X-ray powder diffraction pattern of the crystal form CSV comprises a characteristic peak at a diffraction angle 2θ value of 1, or 2 of 18.1°±0.2°, and 20.0°±0.2° using Cu-Kα radiation; and preferably, the X-ray powder diffraction pattern of the crystal form CSV comprises characteristic peaks at diffraction angle 2θ values of 18.1°±0.2°, and 20.0°±0.2°.

Further, the X-ray powder diffraction pattern of the crystal form CSV comprises a characteristic peak at a diffraction angle 2θ value of 1, or 2 of 20.6°±0.2°, and 12.5°±0.2° using Cu-Kα radiation; and preferably, the X-ray powder diffraction pattern of the crystal form CSV comprises characteristic peaks at diffraction angle 2θ values of 20.6°±0.2°, and 12.5°±0.2°.

In another aspect, the X-ray powder diffraction pattern of the crystal form CSV comprises a characteristic peak at a diffraction angle 2θ value of any 1, or 2, or 3, or 4, or 5, or 6, or 7, or 8, or 9 of 10.3°±0.2°, 19.2°±0.2°, 13.5°±0.2°, 14.5°±0.2°, 18.1°±0.2°, 20.0°±0.2°, 20.6°±0.2°, 12.5°±0.2°, 11.5°±0.2°, 24.9°±0.2°, 25.4°±0.2°, 22.5°±0.2°, 22.0°±0.2°, 23.2°±0.2°, and 30.7°±0.2°.

Without limitation, the X-ray powder diffraction pattern of the crystal form CSV is substantially as shown in FIG. 9 using Cu-Kα radiation.

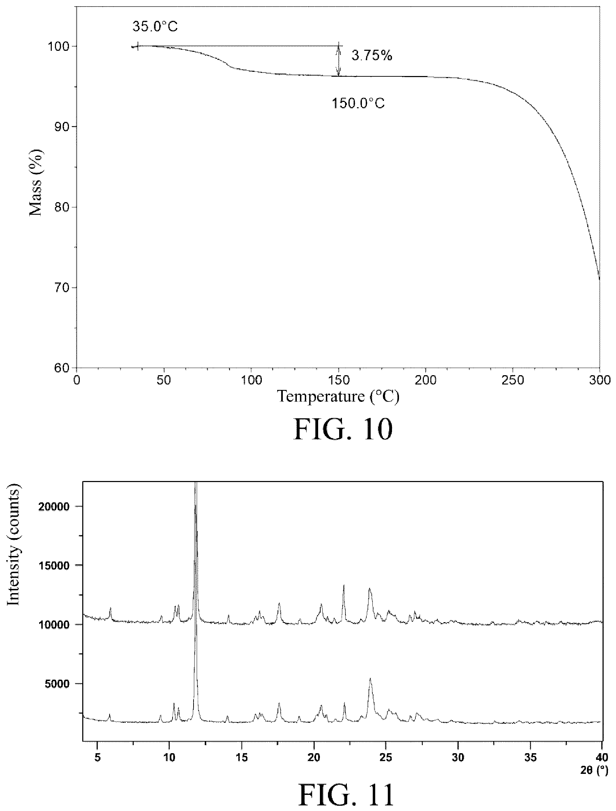

Without limitation, a thermo gravimetric analysis thermogram of the crystal form CSV is substantially as shown in FIG. 10 with a weight loss of about 3.8% when heated from 35°C to 150°C.

According to an object of the present invention, the present invention also provides a method for preparing the crystal form CSV, the method comprising: placing Compound I in a mixed system of dimethyl sulfoxide and water, and stirring the same to obtain the crystal form CSV.

Further, the stirring temperature is preferably 0°C-50°C, more preferably 25°C-50°C. The stirring time is preferably 1-10 day, further preferably 4-6 day.

According to an object of the present invention, the present invention provides the use of the crystal forms CSI, CSII, CSIII, CSIV and/or CSV in preparation of other crystal forms or co-crystals of Compound I and salts thereof.

Further, the present invention provides a pharmaceutical composition comprising a therapeutically effective amount of the crystal forms CSI, CSII, CSIII, CSIV and/or CSV, and a pharmaceutically acceptable excipient.

Further, according to an object of the present invention, the present invention provides the use of the crystal forms CSI, CSII, CSIII, CSIV and/or CSV in preparation of an M4 receptor modulator drug.

Further, according to an object of the present invention, the present invention provides the use of the crystal forms CSI, CSII, CSIII, CSIV and/or CSV in preparation of a drug for the treatment of schizophrenia.

### Advantageous Property of Crystal Form Provided by the Invention

The crystal form CSI provided by the present invention has the following excellent properties.
(1) The crystal form CSI drug substance and formulation provided in the present invention both have good physicochemical stability. The crystal form CSI drug substance provided in the present invention is stable for at least 6 months under the condition of 40°C/75% RH, and has substantially unchanged purity during storage; and a drug formulation formed by mixing the crystal form CSI of the present invention with excipients is stable for at least 1 month under the conditions of 25°C/60% RH, and 40°C/75% RH, and has substantially unchanged purity during storage; and the crystal form CSI drug substance shows no significant change in sample properties and no change in the crystal form after the humidity cycle of 30% -90% -0% -90% RH.
   High temperature and high humidity conditions, caused by seasonal differences, climate differences in different regions, environmental factors, etc., may affect storage, transportation, and production of the drug substance and formulations. The crystal form CSI drug substance and formulation have good physicochemical stability, which is useful to avoid the impact on drug quality due to crystal transformation or decrease in purity during drug storage.
(2) The crystal form CSI provided by the present invention exhibits a small weight increase due to hygroscopicity. The weight increase of the crystal form CSI due to hygroscopicity is 0.36% at the condition of 30%-80% RH. Strong hygroscopicity tends to cause chemical degradation and crystal transformation of the drug substance, thereby affecting physicochemical stability of the drug substance. At the same time, a drug with strong hygroscopicity places higher demands on the production. The crystal form with low hygroscopicity requires a less demanding environment, thereby reducing the cost for material production, preservation and quality control, and is economically valuable.
(3) The crystal form CSI provided by the present invention exhibits good physical stability under mechanical force. The crystal form of the crystal form CSI drug substance remains unchanged after grinding and formulation formula processes. Drug substances are often ground or pulverized during formulation processing, and good physical stability can reduce the risks of reduced crystallinity and crystal transformation of the drug substance during formulation processing. In addition, the crystal form CSI drug substance also has good physical stability under pressure, which is useful to keep the crystal form stable during a tableting process for a formulation.

The crystal form CSII provided by the present invention has the following excellent properties.
(1) The crystal form CSII drug substance and formulation provided by the present invention both have good physicochemical stability. The crystal form CSII drug substance is stable for at least 6 months under the condition of 25°C/60% RH, and 40°C/75% RH, and at least 1 month under the condition of 60°C/75% RH, and has substantially unchanged purity during storage; and a drug formulation formed by mixing the crystal form CSII of the present invention with excipients is stable for at least 1 month under the conditions of 25°C/60% RH, and 40°C/75% RH, and has the substantially unchanged purity during storage; and the crystal form CSII drug substance shows no significant change in sample properties and no change in the crystal form after the humidity cycle of 0% -90% -0% RH.
   High temperature and high humidity conditions, caused by seasonal differences, climate differences in different regions, environmental factors, etc., may affect storage, transportation, and production of the drug substances and formulations. The crystal form CSII drug substance and formulation have good physicochemical stability, which is useful to avoid the impact on drug quality due to crystal transformation or decrease in purity during drug storage.
(2) The crystal form CSII provided by the present invention exhibits a small weight increase due to hygroscopicity. The weight increase of the crystal form CSII due to hygroscopicity is 0.28% under the condition of 0%-80% RH. Strong hygroscopicity tends to cause chemical degradation and crystal transformation of the drug substance, thereby affecting physicochemical stability of the drug substance. At the same time, a drug with strong hygroscopicity places higher demands on the production. The crystal form with low hygroscopicity requires a less demanding environment, thereby reducing the cost for material production, preservation and quality control, and is economically valuable.
(3) The crystal form CSII provided by the present invention exhibits good physical stability under mechanical force. The crystal form of the crystal form CSII drug substance remains unchanged after grinding and formulation formula processes. Drug substances are often ground or pulverized during formulation processing, and good physical stability can reduce the risks of reduced crystallinity and crystal transformation of the drug substance during formulation processing. In addition, the crystal form CSII drug substance also has good physical stability under pressure, which is useful to keep the crystal form stable during a tableting process for a formulation.

The crystal form CSIII provided by the present invention has the following excellent properties.
(1) The crystal form CSIII drug substance provided by the present invention has good physicochemical stability. The crystal form CSIII drug substance provided by the present invention is stable for at least 2 months under the condition of 40°C/75% RH. The crystal form CSI drug substance shows no significant change in sample properties and no change in the crystal form after the humidity cycle of 0%-90%-0% RH.
   High temperature and high humidity conditions, caused by seasonal differences, climate differences in different regions, environmental factors, etc., may affect storage, transportation, and production of the drug substance and formulations. The crystal form CSIII drug substance has good physicochemical stability, which is useful to avoid the impact on drug quality due to crystal transformation or decrease in purity during drug storage.
(2) The crystal form CSIII provided by the present invention exhibits a small weight increase due to hygroscopicity. The weight increase of the crystal form CSIII due to hygroscopicity is 0.21% under the condition of 0%-80% RH. Strong hygroscopicity tends to cause chemical degradation and crystal transformation of the drug substance, thereby affecting physicochemical stability of the drug substance. At the same time, a drug with strong hygroscopicity places higher demands on the production. The crystal form with low hygroscopicity requires a less demanding environment, thereby reducing the cost for material production, preservation and quality control, and is economically valuable.
(3) The crystal form CSIII provided by the present invention exhibits good physical stability under mechanical force. The crystal form of the crystal form CSIII drug substance remains unchanged after being ground. Drug substances are often ground or pulverized during formulation processing, and good physical stability can reduce the risks of reduced crystallinity and crystal transformation of the drug substance during formulation processing.

The crystal form CSIV provided by the present invention has the following excellent properties.
(1) The crystal form CSIV drug substance provided by the present invention has good physicochemical stability. The crystal form CSIV drug substance of the present invention is stable for at least 6 months under the conditions of 25°C/60% RH and 40°C/75% RH, and for at least 1 month under the condition of 60°C/75% RH. The crystal form CSIV drug substance shows no significant change in sample properties and no change in the crystal form after the humidity cycle of 0%-90%-0% RH.
   High temperature and high humidity conditions, caused by seasonal differences, climate differences in different regions, environmental factors, etc., may affect storage, transportation, and production of the drug substance and formulations. The crystal form CSIV drug substance has good physicochemical stability, which is useful to avoid the impact on drug quality due to crystal transformation or decrease in purity during drug storage.
(2) The crystal form CSIV provided by the present invention exhibits a small weight increase due to hygroscopicity. The weight increase of crystal form CSIV due to hygroscopicity is 0.11% at 0%-80% RH. Strong hygroscopicity tends to cause chemical degradation and crystal transformation of the drug substance, thereby affecting physicochemical stability of the drug substance. At the same time, a drug with strong hygroscopicity places higher demands on the production. The crystal form with low hygroscopicity requires a less demanding environment, thereby reducing the cost for material production, preservation and quality control, and is economically valuable.
(3) The crystal form CSIV provided by the present invention exhibits good physical stability under mechanical force. The crystal form of the crystal form CSIV drug substance remains unchanged after being ground. Drug substances are often ground or pulverized during formulation processing, and good physical stability can reduce the risks of reduced crystallinity and crystal transformation of the drug substance during formulation processing.

The crystal form CSV provided by the present invention has the following excellent properties.
(1) The crystal form CSV drug substance and formulation provided by the present invention both have good physicochemical stability. The crystal form CSV drug substance of the present invention is stable for at least 6 months under the conditions of 25°C/60% RH and 40°C/75% RH, and for at least 2 month under the condition of 60°C/75% RH; a drug formulation formed by mixing the crystal form CSV of the present invention with excipients is stable for at least 1 month under the conditions of 25°C/60% RH, and 40°C/75% RH, and has substantially unchanged purity during storage; and the crystal form CSV drug substance shows no significant change in sample properties and no change in the crystal form after the humidity cycle of 30%-90%-0%-90% RH.
   High temperature and high humidity conditions, caused by seasonal differences, climate differences in different regions, environmental factors, etc., may affect storage, transportation, and production of the drug substance and formulations. The crystal form CSV drug substance has good physicochemical stability, which is useful to avoid the impact on drug quality due to crystal transformation or decrease in purity during drug storage.
(2) The crystal form CSV provided by the present invention exhibits a small weight increase due to hygroscopicity. The weight increase of crystal form CSV due to hygroscopicity is 0.29% at 30% -80% RH. Strong hygroscopicity tends to cause chemical degradation and crystal transformation of the drug substance, thereby affecting physicochemical stability of the drug substance. At the same time, a drug with strong hygroscopicity places higher demands on the production. The crystal form with low hygroscopicity requires a less demanding environment, thereby reducing the cost for material production, preservation and quality control, and is economically valuable.
(3) The crystal form CSV provided by the present invention exhibits good physical stability under mechanical force. The crystal form of the crystal form CSV drug substance remains unchanged after grinding and formulation formula processes. Drug substances are often ground or pulverized during formulation processing, and good physical stability can reduce the risks of reduced crystallinity and crystal transformation of the drug substance during formulation processing. In addition, the crystal form CSV drug substance also has good physical stability under pressure, which is useful to keep the crystal form stable during a tableting process for a formulation.

### BRIEF DESCRIPTION OF THE DRAWINGS

FIG. 1 shows an XRPD pattern of a crystal form CSI
FIG. 2 shows an XRPD pattern of a crystal form CSII
FIG. 3 shows a TGA thermogram of the crystal form CSII
FIG. 4 shows a DSC thermogram of the crystal form CSII
FIG. 5 shows an XRPD pattern of a crystal form CSIII
FIG. 6 shows a TGA thermogram of the crystal form CSIII
FIG. 7 shows an XRPD pattern of a crystal form CSIV
FIG. 8 shows a TGA thermogram of a crystal form CSIV
FIG. 9 shows an XRPD pattern of a crystal form CSV
FIG. 10 shows a TGA thermogram of a crystal form CSV
FIG. 11 shows a comparison of XRPD patterns of the crystal form CSI before and after storage under the condition of 40°C/75% RH for a certain period of time (from bottom to top: the pattern before storage, and the pattern after storage at 40°C/75% RH for 6 months (open package))
FIG. 12 shows a comparison of XRPD patterns of the crystal form CSI before and after ball milling (from bottom to top: the pattern before ball milling, and the pattern after ball milling).
FIG. 13 shows a DVS pattern of the crystal form CSI
FIG. 14 shows XRPD patterns of the crystal form CSI and a formulation (from bottom to top: the pattern of a crystal form CSI drug substance, the pattern of the crystal form CSI after the formulation process, and the pattern after mixture of excipients and formulation processes)
FIG. 15 shows a comparison of XRPD patterns of a crystal-form-CSI formulation stored under different conditions (from bottom to top: the pattern before storage, the pattern after storage at 25°C/60% RH for 1 month, and the pattern after storage at 40°C/75% RH for 1 month)
FIG. 16 shows a comparison of XRPD patterns of the crystal form CSII before and after storage for a period of time under different conditions (from bottom to top: the pattern before storage, the pattern after storage for 6 months at 25°C/60% RH, the pattern after storage for 6 months at 40°C/75% RH, and the pattern after storage for 1 month at 60°C/75% RH)
FIG. 17 shows a comparison of XRPD patterns of the crystal form CSII before and after ball milling (from bottom to top: the pattern before ball milling, and the pattern after ball milling).
FIG. 18 shows a DVS pattern of the crystal form CSII
FIG. 19 shows XRPD patterns of the crystal form CSII and a formulation (from bottom to top: the pattern of a crystal form CSII drug substance, the pattern of the crystal form CSII after the formulation process, and the pattern after mixture of excipients and formulation processes)
FIG. 20 shows a comparison of XRPD patterns of a crystal form CSII formulation stored under different conditions (from bottom to top: the pattern before storage, the pattern after storage at 25°C/60% RH for 1 month, and the pattern after storage at 40°C/75% RH for 1 month)
FIG. 21 shows a comparison of XRPD patterns of the crystal form CSIII before and after storage at 40°C/75% RH for a certain period of time (from bottom to top: the pattern before storage, and the pattern after storage at 40°C/75% RH for 2 months (sealed package))
FIG. 22 shows a comparison of XRPD patterns of the crystal form CSIII before and after ball milling (from bottom to top: the pattern before ball milling, and the pattern after ball milling).
FIG. 23 shows a DVS pattern of the crystal form CSIII
FIG. 24 shows a comparison of XRPD patterns of the crystal form CSIV before and after storage for a period of time under different conditions (from bottom to top: the pattern before storage, the pattern after storage for 6 months at 25°C/60% RH, the pattern after storage for 6 months at 40°C/75% RH, and the pattern after storage for 1 month at 60°C/75% RH)
FIG. 25 shows a comparison of XRPD patterns of the crystal form CSIV before and after ball milling (from bottom to top: the pattern before ball milling, and the pattern after ball milling).
FIG. 26 shows a DVS pattern of the crystal form CSIV
FIG. 27 shows a comparison of XRPD patterns of the crystal form CSV before and after storage for a period of time under different conditions ((from bottom to top: the pattern before storage, the pattern after storage for 6 months at 25°C/60% RH, the pattern after storage for 6 months at 40°C/75% RH, and the pattern after storage for 2 month at 60°C/75% RH (sealed package))
FIG. 28 shows a comparison of XRPD patterns of the crystal form CSV before and after ball milling (from bottom to top: the pattern before ball milling, and the pattern after ball milling).
FIG. 29 shows a DVS pattern of the crystal form CSV
FIG. 30 shows XRPD patterns of the crystal form CSV and a formulation (from bottom to top: the pattern of a crystal form CSV drug substance, the pattern of the crystal form CSI after the formulation process, and the pattern after mixture of excipients and formulation processes)
FIG. 31 shows a comparison of XRPD patterns of a crystal form CSV formulation stored under different conditions (from bottom to top: the pattern before storage, the pattern after storage at 25°C/60% RH for 1 month, and the pattern after storage at 40°C/75% RH for 1 month)

### DETAILED DESCRIPTION OF THE EMBODIMENTS

The present invention is described in detail with reference to the following embodiments, which describe in detail the methods for preparing and using the crystal form of the present invention. It is obvious to those skilled in the art that many modifications, both to materials and methods, may be implemented without departing from the scope of the invention.

Abbreviations used in the present invention are explained as follows:
XRPD: X-ray Powder Diffraction
TGA: Thermal Gravimetric Analysis
DSC: Differential Scanning Calorimetry
DVS: Dynamic Vapor Sorption
HPLC: High Performance Liquid Chromatography
RH: Relative Humidity

### Instrument and Method for Collecting Data:

The X-ray powder diffraction pattern described in the invention is collected by a Bruker X-ray powder diffractometer. The parameters for the X-ray powder diffraction method according to the invention are as follows:
X-ray light source: Cu, Kα,
Kα1 (Å): 1.54060; Kα2 (Å): 1.54439,
Kα2/Kα1 intensity ratio: 0.50.

The thermal gravimetric analysis (TGA) pattern as described in the invention is collected by TA Q500. The parameters for the thermal gravimetric analysis (TGA) method described in the invention are as follows:
scanning speed: 10°C/min,
protective gas: N₂.

The differential scanning calorimetry (DSC) pattern as described in the present invention is collected by TA Q2000. The parameters for the differential scanning calorimetry (DSC) method described in the present invention are as follows:
scanning speed: 10°C/min,
protective gas: N₂.

The dynamic vapor sorption (DVS) pattern for the experiment is collected by an Intrinsic dynamic vapor sorption instrument manufactured by Surface Measurement Systems Ltd. (SMS). Control software for the instrument is DVS-Intrinsic control software. The method parameters of the dynamic vapor sorption instrument are as follows:
temperature: 25°C,
carrier gas, and flow rate: N₂, and 200 ml/min,
mass change per unit time: 0.002%/min,
relative humidity range: 0%RH-95%RH.

The test methods for related substances in the present invention are shown in table 1.

**Table 1**

| | | |
|---|---|---|
| HPLC | Agilent 1260 | |
| Chromatographic Column | Waters XBridge C18, 4.6×150 mm, 3.5µm | |
| Mobile Phase | A: 0.1% TFA in H2O | |
| | B: 0.1% TFA in ACN | |
| Gradient | Time (min) | % A |
| | 0.0 | 5 |
| | 0.5 | 5 |
| | 15.0 | 30 |
| | 20.0 | 80 |
| | 25.0 | 80 |
| | 25.1 | 5 |
| | 35.0 | 5 |
| Flow Rate | 0.8 mL/min, 1.0 mL/min | |
| Injection Volume | 5 µL | |
| Detection Wavelength | 284 nm | |
| Column Temperature | 40°C/45°C | |
| Diluent | MeOH | |

In the present invention, the "drying" is performed by a conventional method in the art, for example, vacuum drying, air blast drying, or air drying. Drying temperature may be room temperature or higher, preferably room temperature to about 60 °C, or to 50°C, or to 40°C. Drying time may be 2-48 h, or overnight. Drying is carried out in a fume hood, a forced air oven or a vacuum oven.

The "evaporating" is performed by a conventional method in the art, such as slow evaporating or fast evaporating. Slow evaporating is performed in a container sealed with a sealing film with pinholes. Rapid evaporating is performed in an open container.

The "room temperature", which is not a specific temperature value, means a temperature range of 10-30°C.

The "stirring" is performed by a conventional method in the art, for example, magnetic stirring or mechanical stirring, at a stirring speed of 50-1800 rpm, wherein the magnetic stirring speed is preferably 300-900 rpm, and the mechanical stirring speed is preferably 100-300 rpm.

The "separation" is performed by a conventional method in the art, such as centrifugation or filtration. The "centrifugation" comprises the following operations: placing a sample to be subjected to separation in a centrifuge tube and performing centrifugation at 10,000 rpm until all solids sink to the bottom of the centrifuge tube.

The "characteristic peak" refers to a representative diffraction peak used to distinguish crystals, and the peak position may typically have a deviation of ± 0.2° when using Cu-Kα radiation.

In the present invention, an amorphous form refers to a non-crystalline material which possesses no long-range order. Typically, an amorphous form exhibits a broad "halo" X-ray powder diffraction pattern.

In the present invention, "crystal" or "crystal form" can be characterized by X-ray powder diffraction. Those skilled in the art can appreciate that the X-ray powder diffraction pattern may vary depending on an instrument condition, and the preparation and purity of a sample. Relative intensities of diffraction peaks in an X-ray powder diffraction pattern may also vary with experimental conditions, so that the diffraction peak intensity is not the only or determining factor to determine a crystal form. In fact, the relative intensities of the diffraction peaks in an X-ray powder diffraction pattern are related to a preferred orientation of a crystal, and the intensities of the diffraction peaks shown in the present invention are illustrative, not for absolute comparison. Thus, it will be understood by those skilled in the art that the X-ray powder diffraction pattern of the claimed crystal form of the invention needs not be exactly the same as the X-ray powder diffraction patterns in the embodiments herein, and that any crystal form having an X-ray powder diffraction pattern with a characteristic peak that is the same as or similar to that in these patterns is within the scope of the invention. Those skilled in the art can compare the X-ray powder diffraction pattern listed in the present invention with an X-ray powder diffraction pattern of an unknown crystal form to confirm whether the two patterns reflect the same or different crystal forms.

In some embodiments, crystal forms CSI, CSII, CSIII, CSIV, and CSV in the present invention are pure and substantially free of any other crystal forms. As used herein, when used to refer to a novel crystal form, "substantially free" means that the crystal form contains less than 20% (by weight) of other crystal forms, particularly less than 10% (by weight), more particularly less than 5% (by weight), and even more particularly less than 1% (by weight).

As used herein, when used to refer to a measurable numerical value, e.g., mass, time, temperature, etc., the term "about" is meant to encompass variations of ±10%, ±5%, ±1%, ±0.5%, or ±0.1% of the specific numerical value.

The following examples are carried out at room temperature unless otherwise specified.

According to the present invention, compound I as a raw material may be in forms including, but not limited to, a solid form (crystal or amorphous), an oil form, a liquid form, and a solution. Preferably, compound I as a raw material is in a solid form.

Compound I used in the following examples can be prepared according to the prior art, for example, the method described in WO 2018002760 A1, in combination with conventional salt breaking methods in the art.

### Example 1 Preparation of Crystal Form CSI

9.5 mg of compound I was weighed and placed into a vial, and 0.4 mL of acetone was added to obtain a clear solution. The solution was filtered, and the filtrate was evaporated at room temperature to obtain a solid, which was dried under vacuum at 25°C for about 20 hours to obtain a crystalline solid.

Upon testing, the obtained dry solid was the crystal form CSI of the present invention, the X-ray powder diffraction pattern of which is shown in FIG. 1, and the X-ray powder diffraction data is shown in Table 2.

**Table 2**

| Diffraction Angle 2θ (°) | d Value (Å) | Relative Intensity (%) |
|---|---|---|
| 5.9 | 14.9 | 3.8 |
| 9.5 | 9.3 | 2.3 |
| 10.4 | 8.5 | 8.7 |
| 10.7 | 8.3 | 4.0 |
| 11.9 | 7.4 | 100.0 |
| 14.1 | 6.3 | 3.1 |
| 16.0 | 5.5 | 1.1 |
| 16.3 | 5.4 | 3.5 |
| 16.5 | 5.4 | 1.4 |
| 17.7 | 5.0 | 3.5 |
| 19.1 | 4.6 | 2.2 |
| 20.3 | 4.4 | 1.7 |
| 20.6 | 4.3 | 3.9 |
| 21.0 | 4.2 | 4.0 |
| 21.5 | 4.1 | 1.4 |
| 22.2 | 4.0 | 9.0 |
| 23.9 | 3.7 | 4.8 |
| 24.1 | 3.7 | 3.2 |
| 24.5 | 3.6 | 2.6 |
| 25.3 | 3.5 | 1.7 |
| 25.5 | 3.5 | 1.7 |
| 25.7 | 3.5 | 1.1 |
| 26.7 | 3.3 | 2.7 |
| 27.1 | 3.3 | 3.5 |
| 34.3 | 2.6 | 1.1 |
| 37.2 | 2.4 | 1.2 |

### Example 2 Preparation of Crystal Form CSII

9.1 mg of compound I was weighed and placed into a vial, 0.2 mL of a mixed solvent of methanol/methyl tert-butyl ether (1:9, v/v) was added thereto, followed by stirring at room temperature for about 68 hours, and a solid was separated by centrifugation and dried under vacuum at 25°C for about 20 hours to obtain a crystalline solid.

Upon testing, the obtained dry solid was the crystal form CSII of the present invention, the X-ray powder diffraction pattern of which is shown in FIG. 2, and the X-ray powder diffraction data is shown in Table 3.

TGA is as shown in FIG. 3, with substantially no weight loss upon heating from room temperature to 180°C.

DSC is as shown in FIG. 4, with an endothermic peak appearing at about 183°C, which is a melting endothermic peak.

**Table 3**

| Diffraction Angle 2θ (°) | d Value (Å) | Relative Intensity (%) |
|---|---|---|
| 6.4 | 13.9 | 3.9 |
| 9.0 | 9.9 | 1.4 |
| 11.4 | 7.8 | 2.8 |
| 11.6 | 7.6 | 2.0 |
| 11.7 | 7.5 | 2.3 |
| 12.8 | 6.9 | 100.0 |
| 13.0 | 6.8 | 9.5 |
| 14.3 | 6.2 | 2.4 |
| 14.6 | 6.1 | 4.1 |
| 15.0 | 5.9 | 5.1 |
| 15.2 | 5.8 | 2.6 |
| 15.9 | 5.6 | 1.9 |
| 16.3 | 5.5 | 3.9 |
| 16.9 | 5.3 | 1.1 |
| 18.0 | 4.9 | 2.4 |
| 18.1 | 4.9 | 4.8 |
| 19.2 | 4.6 | 6.6 |
| 19.8 | 4.5 | 11.9 |
| 20.3 | 4.4 | 3.1 |
| 20.8 | 4.3 | 6.0 |
| 21.5 | 4.1 | 9.3 |
| 22.5 | 4.0 | 12.8 |
| 23.3 | 3.8 | 13.3 |
| 23.6 | 3.8 | 2.9 |
| 24.1 | 3.7 | 4.4 |
| 24.4 | 3.6 | 25.8 |
| 25.4 | 3.5 | 2.1 |
| 25.8 | 3.5 | 6.2 |
| 27.1 | 3.3 | 4.3 |
| 27.8 | 3.2 | 4.4 |
| 28.3 | 3.1 | 2.9 |
| 39.0 | 2.3 | 1.0 |

### Example 3 Preparation of Crystal Form CSIII

14.6 mg of compound I was weighed and placed into a vial, and 2 mL of a mixed solvent of tetrahydrofuran/n-heptane (1:1, v/v) was added to obtain a clear solution. The solution was filtered, and a half of the filtrate was taken and evaporated at 50°C to obtain a solid, which was dried under vacuum at room temperature for about 24 hours to obtain a crystalline solid. Upon testing, the obtained crystalline solid was the crystal form CSIII of the present invention, the X-ray powder diffraction pattern of which is shown in FIG. 5, and the X-ray powder diffraction data is shown in Table 4.

TGA is as shown in FIG. 6, with substantially no weight loss upon heating from room temperature to 170°C.

**Table 4**

| Diffraction Angle 2θ (°) | d Value (Å) | Relative Intensity (%) |
|---|---|---|
| 6.2 | 14.2 | 23.4 |
| 10.0 | 8.9 | 1.6 |
| 11.6 | 7.6 | 6.2 |
| 12.5 | 7.1 | 100.0 |
| 12.7 | 7.0 | 23.1 |
| 13.6 | 6.5 | 3.0 |
| 15.6 | 5.7 | 3.6 |
| 16.5 | 5.4 | 4.6 |
| 17.5 | 5.1 | 5.2 |
| 18.1 | 4.9 | 5.3 |
| 18.8 | 4.7 | 14.9 |
| 20.5 | 4.3 | 1.7 |
| 21.7 | 4.1 | 5.9 |
| 22.5 | 3.9 | 2.0 |
| 24.2 | 3.7 | 6.6 |
| 25.5 | 3.5 | 2.3 |
| 27.0 | 3.3 | 1.8 |
| 27.3 | 3.3 | 1.7 |
| 34.4 | 2.6 | 0.3 |
| 38.1 | 2.4 | 1.1 |

### Example 4 Preparation of Crystal Form CSIV

Compound I was heated to 190°C in protective nitrogen atmosphere at 10°C/min, and was held at the temperature for 1 minute to obtain a crystalline solid. Upon testing, the obtained crystalline solid was the crystal form CSIV of the present invention, the X-ray powder diffraction pattern of which is shown in FIG. 7, and the X-ray powder diffraction data of which is shown in Table 5.

TGA is as shown in FIG. 8, with substantially no weight loss upon heating from room temperature to 180°C.

**Table 5**

| Diffraction Angle 2θ (°) | d Value (Å) | Relative Intensity (%) |
|---|---|---|
| 6.5 | 13.6 | 2.0 |
| 8.6 | 10.3 | 2.1 |
| 11.3 | 7.9 | 2.8 |
| 11.5 | 7.7 | 9.0 |
| 13.0 | 6.8 | 100.0 |
| 14.2 | 6.3 | 24.8 |
| 16.4 | 5.4 | 32.8 |
| 17.2 | 5.1 | 22.0 |
| 18.1 | 4.9 | 9.2 |
| 19.6 | 4.5 | 18.5 |
| 19.7 | 4.5 | 17.8 |
| 20.0 | 4.4 | 26.1 |
| 20.4 | 4.4 | 22.8 |
| 20.8 | 4.3 | 16.6 |
| 21.4 | 4.2 | 3.5 |
| 21.9 | 4.1 | 8.4 |
| 22.0 | 4.0 | 9.2 |
| 22.6 | 3.9 | 8.1 |
| 23.1 | 3.9 | 38.7 |
| 24.0 | 3.7 | 52.1 |
| 24.8 | 3.6 | 4.3 |
| 26.4 | 3.4 | 11.7 |
| 26.5 | 3.4 | 7.5 |
| 27.2 | 3.3 | 9.9 |
| 27.9 | 3.2 | 3.7 |
| 28.1 | 3.2 | 4.9 |
| 28.7 | 3.1 | 3.9 |
| 29.5 | 3.0 | 2.0 |
| 30.4 | 2.9 | 3.6 |
| 33.0 | 2.7 | 3.1 |
| 35.6 | 2.5 | 2.0 |

### Example 5 Preparation of Crystal Form CSV

3.8 mg of compound I was weighed and placed into a vial, 0.1 mL of a mixed solvent of dimethyl sulfoxide/water (1:1, v/v) was added, and the mixture was stirred at 50°C for 4 days to obtain a crystalline solid. Upon testing, the obtained crystalline solid was the crystal form CSV of the present invention, the X-ray powder diffraction pattern of which is shown in FIG. 9, and the X-ray powder diffraction data is shown in Table 6.

**Table 6**

| Diffraction Angle 2θ (°) | d Value (Å) | Relative Intensity (%) |
|---|---|---|
| 10.3 | 8.6 | 53.4 |
| 11.5 | 7.7 | 100.0 |
| 12.5 | 7.1 | 14.2 |
| 13.1 | 6.7 | 4.9 |
| 13.5 | 6.6 | 17.3 |
| 14.5 | 6.1 | 23.7 |
| 15.2 | 5.8 | 5.3 |
| 16.8 | 5.3 | 11.3 |
| 17.4 | 5.1 | 16.8 |
| 17.5 | 5.1 | 35.9 |
| 18.1 | 4.9 | 28.1 |
| 19.2 | 4.6 | 39.9 |
| 20.0 | 4.4 | 28.9 |
| 20.6 | 4.3 | 23.9 |
| 21.3 | 4.2 | 7.8 |
| 21.6 | 4.1 | 5.6 |
| 22.0 | 4.0 | 14.9 |
| 22.5 | 4.0 | 17.1 |
| 23.2 | 3.8 | 20.8 |
| 24.2 | 3.7 | 5.5 |
| 24.6 | 3.6 | 19.1 |
| 24.9 | 3.6 | 60.9 |
| 25.2 | 3.5 | 15.1 |
| 25.4 | 3.5 | 33.4 |
| 26.1 | 3.4 | 9.2 |
| 26.7 | 3.3 | 12.3 |
| 27.2 | 3.3 | 9.5 |
| 28.6 | 3.1 | 8.6 |
| 28.8 | 3.1 | 12.7 |
| 30.7 | 2.9 | 3.3 |
| 34.5 | 2.6 | 1.4 |
| 36.5 | 2.5 | 2.4 |
| 38.2 | 2.4 | 1.5 |
| 39.4 | 2.3 | 3.0 |

### Example 6 TGA of Crystal Form CSV

TGA of crystal form CSV is shown in FIG. 10, which shows that the crystal form CSV has a weight loss of 3.8% when heated from 35°C to 150°C.

### Example 7 Stability of Crystal Form CSI

An appropriate amount of a crystal-form-CSI sample was packaged under corresponding packaging conditions and stored at 40°C/75% RH for a certain period of time, the purity and crystal form were determined by HPLC and XRPD, and the results are shown in Table 7. The XRPD patterns of crystal form CSI before and after storage are shown in FIG. 11.

**Table 7**

| Initial Crystal Form | Storage Condition | Packaging Condition | Storage Time | Crystal Form | Purity |
|---|---|---|---|---|---|
| Crystal Form CSI | Initial | - | - | Crystal Form CSI | 99.53% |
| | 40°C/75% RH | Open | 6 Month | Crystal Form CSI | 99.59% |

Open: a sample was placed in a glass vial without a lid and stored in corresponding environment while the vial was open.

The result shows that crystal form CSI is stable for at least 6 months at 40°C/75% RH, and has good physicochemical stability.

### Example 8 Stability of Crystal Form CSI under Mechanical Force

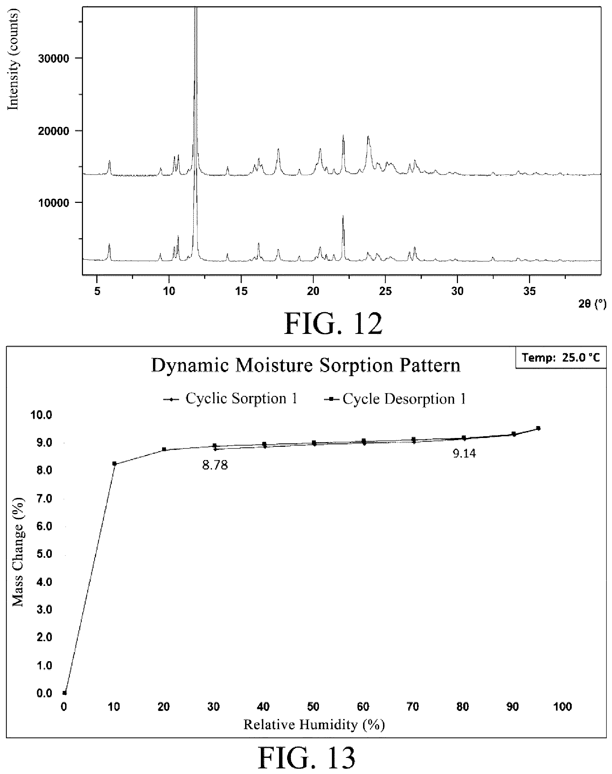

A crystal-form-CSI sample was ball-milled at a vibration speed of 500 rpm for 5 minutes using a ball mill, the sample before and after ball milling was tested by XRPD, the test result is shown in FIG. 12, and crystal form CSI remains unchanged after ball milling.

An appropriate amount of crystal form CSI was taken, compressed into a tablet using a manual tablet press under a pressure of 15 KN with a φ 6 mm round tooling, XRPD tests were performed before and after tableting, and the test results show that crystal form CSI remains unchanged after tableting.

The result shows that crystal form CSI has good stability under mechanical force.

### Example 9 Hygroscopicity of Crystal Form CSI

About 10 mg of a crystal-form-CSI sample was taken and tested for hygroscopicity using a dynamic vapor sorption (DVS) instrument, with a relative humidity cycle of 30%-90%-0%-90% RH, and a mass change for each humidity was recorded. The sample was tested by XRPD before and after DVS testing. The experimental result shows that crystal form CSI remains unchanged after DVS testing. The DVS pattern of crystal form CSI is shown in FIG. 13, and the weight increase of crystal form CSI due to hygroscopicity is 0.36% at 30%-80% RH.

### Example 10 Preparation of Crystal-Form-CSI Formulation

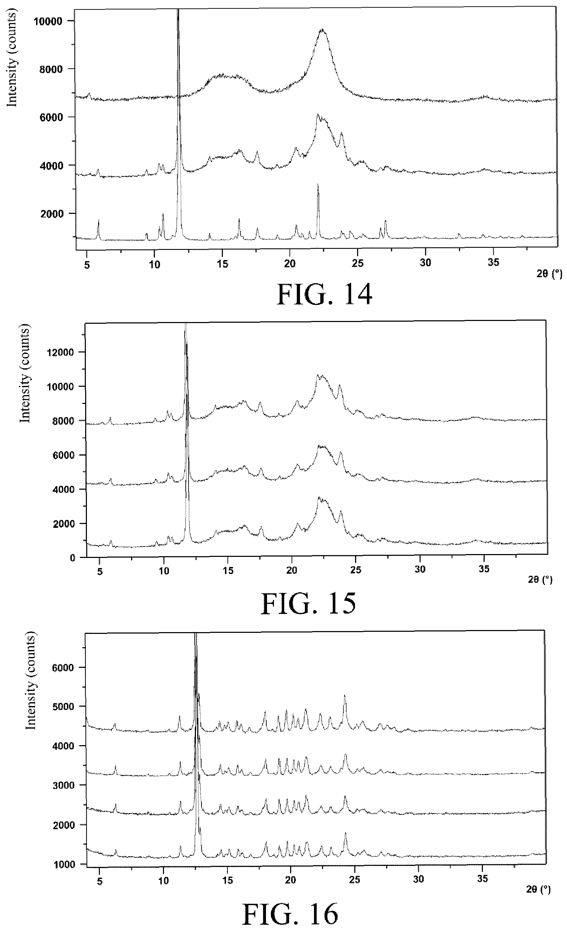

A crystal-form-CSI formulation was prepared by using the formulation formula shown in table 8 and the formulation process shown in table 10, and a formula for mixture of excipients is shown in table 9. A mixture of excipients and the sample before and after the formulation formulas were tested by XRPD, and the result is shown in FIG. 14. The result shows that crystal form CSI is not changed after the formulation formula processes.

**Table 8**

| | Component | % (w/w) | mg/ Tablet | Function |
|---|---|---|---|---|
| 1 | Compound I | 30.0 | 30.0 | Primary Drug |
| 2 | Microcrystalline Cellulose | 59.5 | 59.5 | Filler |
| 3 | Hydroxypropylmethylcellulose | 3.0 | 3.0 | Binder |
| 4 | Crospovidone | 6.0 | 6.0 | Disintegrant |
| 5 | Micronized Silica Gel | 0.5 | 0.5 | Glidant |
| 6 | Magnesium Stearate | 1.0 | 1.0 | Lubricant |
| In Total | | | 100 | / |

**Table 9**

| Excipients Formula | | % (w/w) | Function |
|---|---|---|---|
| 1 | Microcrystalline Cellulose | 85.0 | Filler |
| 2 | Hydroxypropylmethylcellulose | 4.3 | Binder |
| 3 | Crospovidone | 8.6 | Disintegrant |
| 4 | Micronized Silica Gel | 0.7 | Glidant |
| 5 | Magnesium Stearate | 1.4 | Lubricant |
| In Total | | 100.0 | / |

**Table 10**

| Stage | Step |
|---|---|
| Pre-mixing | weighing No. 1 ~ 6 materials according to the formula and placing them into a glass vial, followed by manual mixing for 10 min; |
| Simulated Dry Granulation | mounting, in a ENERPAC single-punch manual tablet press, a circular punch die with Φ20 mm round die, pressing the premixed powder (100±10 mg) into flakes with a pressure of 10±1 KN, gently crushing the flakes, and passing the crushed flakes through a 20 mesh screen to obtain a final mixture; |
| Tableting | pressing the final mixture (100±10 mg) into a tablet using a pressure of 5±1 KN with a T8 × 4 mm special-shaped die mounted in a ENERPAC single-punch manual tablet press. |
| Packaging | Placing 1 tablet and 1 g of a desiccant in a 35-cc HDPE vial, and sealing the vial. |

### Example 11 Stability of Crystal-Form-CSI Formulation

A sample of a crystal-form-CSI formulation was packaged under corresponding conditions and stored at 25°C/60% RH, the purity and crystal form were determined by HPLC and XRPD. The result is shown in Table 11, and the XRPD patterns before and after storage are shown in FIG. 15. The result shows that the crystal-form-CSI formulation sample is stable for at least 1 month at 25°C/60% RH with little change in purity.

**Table 11**

| Storage Condition | Packaging Condition | Storage Time | Crystal Form | Purity (%) |
|---|---|---|---|---|
| Initial | - | - | Crystal Form CSI | 99.73 |
| 25°C/60% RH | Sealed | 1 Month | Crystal Form CSI | 99.72 |

### Example 12 Stability of Crystal Form CSII

An appropriate amount of a crystal-form-CSII sample was packaged under corresponding packaging conditions and stored for a certain period of time at 25°C/60% RH, 40°C/75% RH, and 60°C/75% RH, the purity and crystal form were determined by HPLC and XRPD, and the result is shown in Table 12. XRPD patterns of crystal form CSII before and after storage are shown in FIG. 16.

**Table 12**

| Initial Crystal Form | Storage Condition | Packaging Condition | Storage Time | Crystal Form | Purity |
|---|---|---|---|---|---|
| Crystal Form CSII | Starting | - | - | Crystal Form CSII | 99.56% |
| | 25°C/60% RH | Sealed | 6 Month | Crystal Form CSII | 99.60% |
| | 40°C/75% RH | Sealed | 6 Month | Crystal Form CSII | 99.57% |
| | 60°C/75% RH | Sealed | 1 Month | Crystal Form CSII | 99.55% |

Sealing: The sample was placed in a glass vial, which was covered by a lid and sealed in a double-layer PE bag, followed by an aluminum foil bag.

The result shows that crystal form CSII is stable for at least 6 months at 25°C/60% RH and 40°C/75% RH, and for at least 1 month at 60°C/75% RH, and has good physicochemical stability.

### Example 13 Stability of CSII Form under Mechanical Force

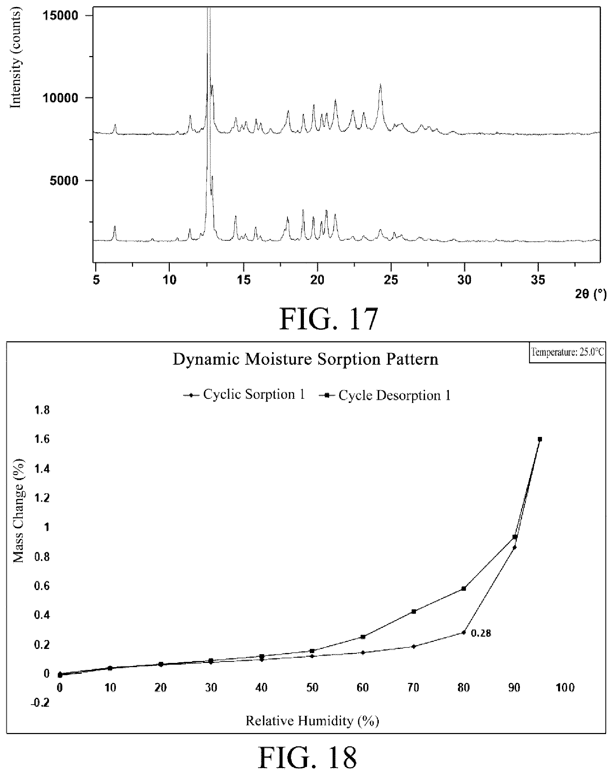

A crystal-form-CSII sample was ball-milled at a vibration speed of 500 rpm for 5 minutes using a ball mill, the sample before and after ball milling was tested by XRPD, the test result is shown in FIG. 17, and crystal form CSII is not changed after ball milling.

An appropriate amount of a crystal-form-CSII sample was taken, pressed into a tablet using a manual tablet press under a pressure of 15 KN with a φ 6 mm round tooling, XRPD tests were performed before and after tableting, and the test results show that crystal form CSII is not changed after tableting.

The result shows that crystal form CSII has good stability under mechanical force.

### Example 14 Hygroscopicity of Crystal Form CSI

About 10 mg of a crystal-form-CSII sample was taken and tested for hygroscopicity using a dynamic vapor sorption (DVS) instrument, with a relative humidity cycle of 0%-90%-0% RH, and a mass change for each humidity was recorded. The sample was tested by XRPD before and after DVS testing. The experimental result shows that crystal form CSII is not changed after DVS testing. The DVS pattern of crystal form CSII is shown in FIG. 18, and the weight increase of crystal form CSII due to hygroscopicity is 0.28% at 0%-80% RH.

### Example 15 Preparation of Crystal-Form-CSII Formulation

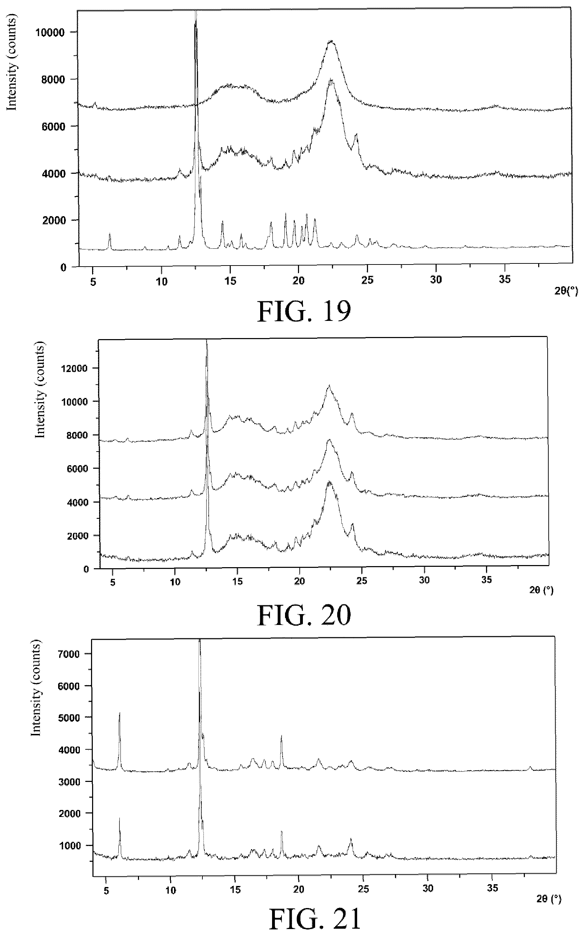

A crystal-form-CSII formulation was prepared by using the formulation formula shown in Table 8 and the formulation process shown in Table 10, and an excipient formula is shown in Table 9. A mixture of excipients and the sample before and after the formulation formulas were tested by XRPD, and the result is shown in FIG. 19. The result shows that crystal form CSII is not changed after the formulation formula processes.

### Example 16 Stability of Crystal-Form-CSII Formulation

A sample of a crystal-form-CSII formulation was packaged under corresponding conditions and stored at 25°C/60% RH and 40°C/75% RH, the purity and crystal form were determined by HPLC and XRPD, the result is shown in Table 13, and the XRPD patterns before and after storage are shown in FIG. 20. The result shows that the crystal-form-CSII formulation sample is stable for at least 1 month at 25°C/60% RH and 40°C/75% RH with little change in purity.

**Table 13**

| Storage Condition | Packaging Condition | Storage Time | Crystal Form | Purity (%) |
|---|---|---|---|---|
| Starting | - | - | Crystal Form CSII | 99.98 |
| 25°C/60% RH | Sealed | 1 Month | Crystal Form CSII | 99.98 |
| 40°C/75% RH | Sealed (With Desiccant) | 1 Month | Crystal Form CSII | 99.98 |

### Example 17 Stability of Crystal Form CSIII

An appropriate amount of a crystal-form-CSIII sample was packaged under corresponding packaging conditions and stored for a certain period of time at 40°C/75% RH, the crystal form was determined by XRPD, and the result is shown in Table 14. XRPD patterns of crystal form CSIII before and after storage are shown in FIG. 21.

**Table 14**

| Initial Crystal Form | Storage Condition | Packaging Condition | Storage Time | Crystal Form |
|---|---|---|---|---|
| Crystal Form CSIII | Initial | - | - | Crystal Form CSIII |
| | 40°C/75% RH | Sealed | 2 Month | Crystal Form CSIII |

Sealed: The sample was placed in a glass vial, which was covered by a lid and sealed in a double-layer PE bag, followed by an aluminum foil bag.

The result shows that crystal form CSIII is stable for at least 2 months at 40°C/75% RH, and has good physical stability.

### Example 18 Stability of Crystal Form CSIII under Mechanical Force

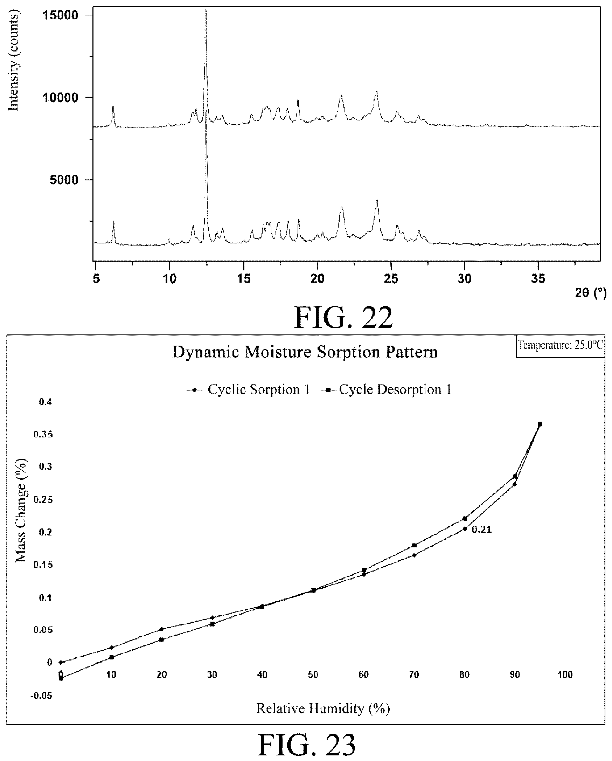

A crystal-form-CSIII sample was ball-milled at a vibration speed of 500 rpm for 5 minutes using a ball mill. The sample before and after ball milling was tested by XRPD, the test result is shown in FIG. 22, and crystal form CSIII is not changed after the ball milling.

The result shows that crystal form CSIII has good stability under mechanical force.

### Example 19 Hygroscopicity of Crystal Form CSIII

About 10 mg of a crystal-form-CSIII sample was taken and tested for hygroscopicity using a dynamic vapor sorption (DVS) instrument, with a relative humidity cycle of 0%-90%-0% RH, and a mass change for each humidity was recorded. The sample was tested by XRPD before and after DVS testing. The experimental result shows that crystal form CSIII is not changed after DVS testing. The DVS pattern of crystal form CSIII is shown in FIG. 23, and the weight increase of crystal form CSIII due to hygroscopicity is 0.21% at 0%-80% RH.

### Example 20 Stability of Crystal Form CSIV

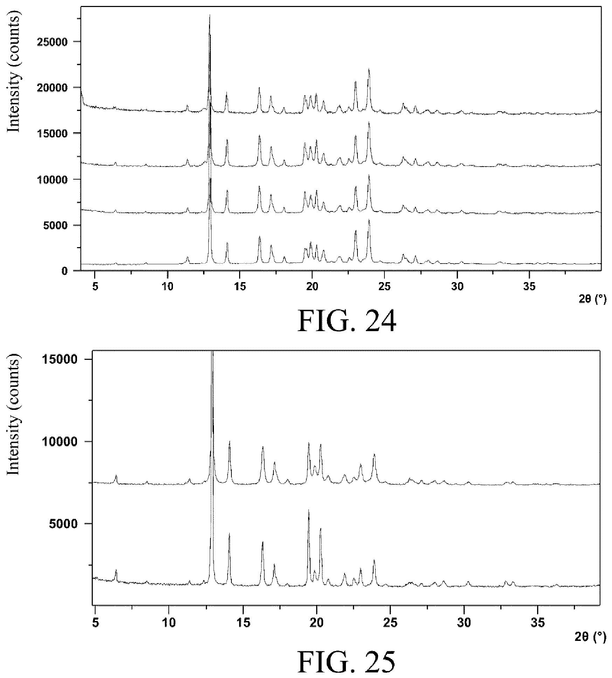

An appropriate amount of a crystal-form-CSIV sample was packaged under corresponding packaging conditions and stored for a certain period of time at 25°C/60% RH, 40°C/75% RH, and 60°C/75% RH, the crystal form was determined by XRPD, and the result is shown in Table 15. XRPD patterns of crystal form CSIV before and after the storage are shown in FIG. 24.

**Table 15**

| Initial Crystal Form | Storage Condition | Packaging Condition | Storage Time | Crystal Form |
|---|---|---|---|---|
| Crystal Form CSIV | Starting | - | - | Crystal Form CSIV |
| | 25°C/60% RH | Sealed | 6 Month | Crystal Form CSIV |
| | 40°C/75% RH | Sealed | 6 Month | Crystal Form CSIV |
| | 60 °C/75% RH | Sealed | 1 Month | Crystal Form CSIV |

Sealed: The sample was placed in a glass vial, which was covered by a lid and sealed in a double-layer PE bag, followed by an aluminum foil bag.

The result shows that crystal form CSIV is stable for at least 6 months at 25°C/60% RH and 40°C/75% RH, and for at least 1 month at 60°C/75% RH, and has good physicochemical stability.

### Example 21 Stability of Crystal Form CSIV under Mechanical Force

A crystal-form-CSIV sample was ball-milled at a vibration speed of 500 rpm for 5 minutes using a ball mill, the sample before and after ball milling was tested by XRPD, the test result is shown in FIG. 25, and crystal form CSIV is not changed after ball milling.

The result shows that crystal form CSIV has good stability under mechanical force.

### Example 22 Hygroscopicity of Crystal Form CSIV

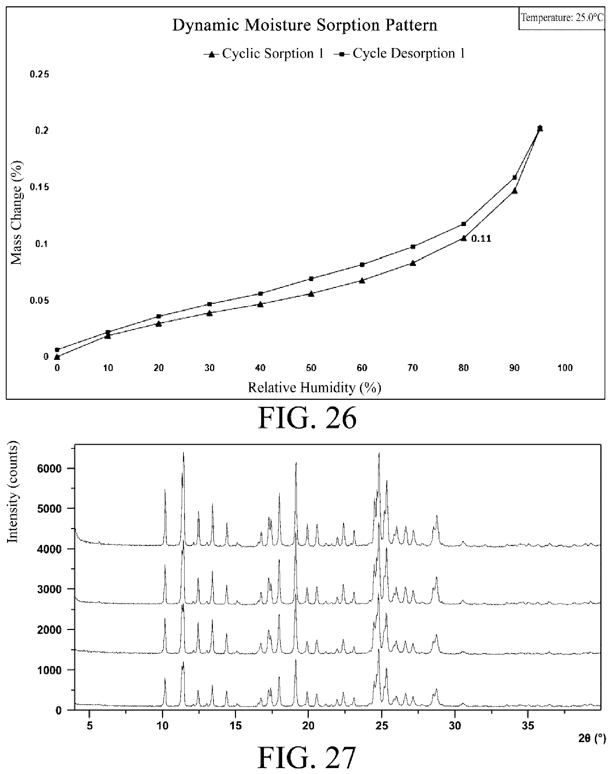

About 10 mg of a crystal-form-CSIV sample was taken and tested for hygroscopicity using a dynamic vapor sorption (DVS) instrument, with a relative humidity cycle of 0%-90%-0% RH, and a mass change for each humidity was recorded. The sample was tested by XRPD before and after DVS testing. The experimental result shows that crystal form CSIV is not changed after DVS testing. The DVS pattern of crystal form CSIV is shown in FIG. 26, and the weight increase of crystal form CSIV due to hygroscopicity is 0.11% at 0%-80% RH.

### Example 23 Stability of Crystal Form CSV

An appropriate amount of a crystal-form-CSV sample was packaged under corresponding packaging conditions and stored for a certain period of time at 25°C/60% RH, 40°C/75% RH, and 60°C/75% RH, the purity and crystal form were determined by HPLC and XRPD, and the result is shown in Table 16. XRPD patterns of crystal form CSV before and after storage are shown in FIG. 27.

**Table 16**

| Initial Crystal Form | Storage Condition | Packaging Condition | Storage Time | Crystal Form | Purity |
|---|---|---|---|---|---|
| Crystal Form CSV | Starting | - | - | Crystal Form CSV | 99.96% |
| | 25°C/60% RH | Sealed | 6 Month | Crystal Form CSV | 99.96% |
| | 40°C/75% RH | Sealed | 6 Month | Crystal Form CSV | 99.96% |
| | 60°C/75% RH | Sealed | 2 Month | Crystal Form CSV | 99.95% |

Sealed: The sample was placed in a glass vial, which was covered by a lid and sealed in a double-layer PE bag, followed by an aluminum foil bag.

The result shows that crystal form CSV is stable for at least 6 months at 25°C/60% RH and 40°C/75% RH, and for at least 2 months at 60°C/75% RH, and has good physicochemical stability.

### Example 24 Stability of Crystal Form CSV under Mechanical Force

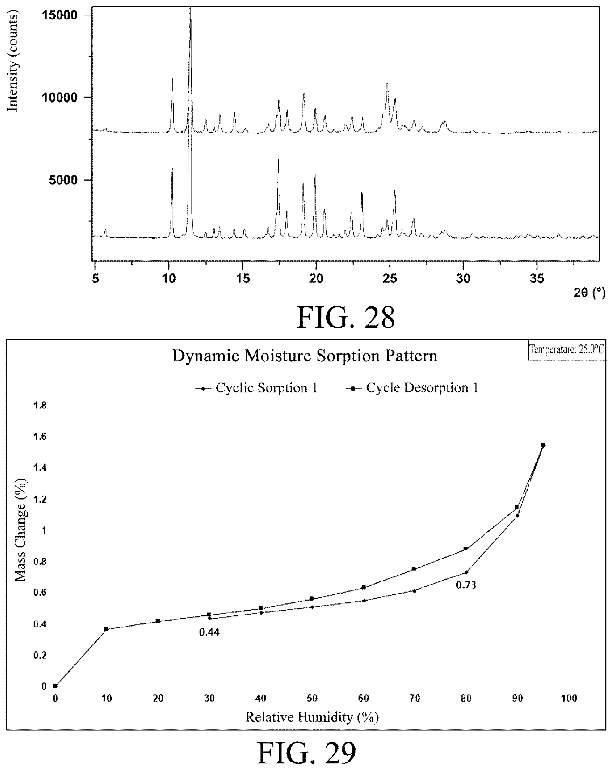

A crystal-form-CSV sample was ball-milled at a vibration speed of 500 rpm for 5 minutes using a ball mill, the sample before and after ball milling was tested by XRPD, the test result is shown in FIG. 28, and crystal form CSV is not changed after ball milling.

An appropriate amount of a crystal-form-CSV sample was taken, pressed into a tablet using a manual tablet press under a pressure of 15 KN with a φ 6 mm round tooling, XRPD tests were performed before and after tableting, and the test results show that crystal form CSV is not changed after tableting.

The result shows that crystal form CSV has good stability under mechanical force.

### Example 25 Hygroscopicity of Crystal Form CSV

About 10 mg of a crystal-form-CSV sample was taken and tested for hygroscopicity using a dynamic vapor sorption (DVS) instrument, with a cycle of 30%-90%-0%-90% RH, and a mass change for each humidity was recorded. The sample was tested by XRPD before and after DVS testing. The experimental result shows that crystal form CSV is not changed after DVS testing. The DVS pattern of crystal form CSV is shown in FIG. 29, and the weight increase of crystal form CSV due to hygroscopicity is 0.29% at 30%-80% RH.

### Example 26 Preparation of Crystal-Form-CSV Formulation

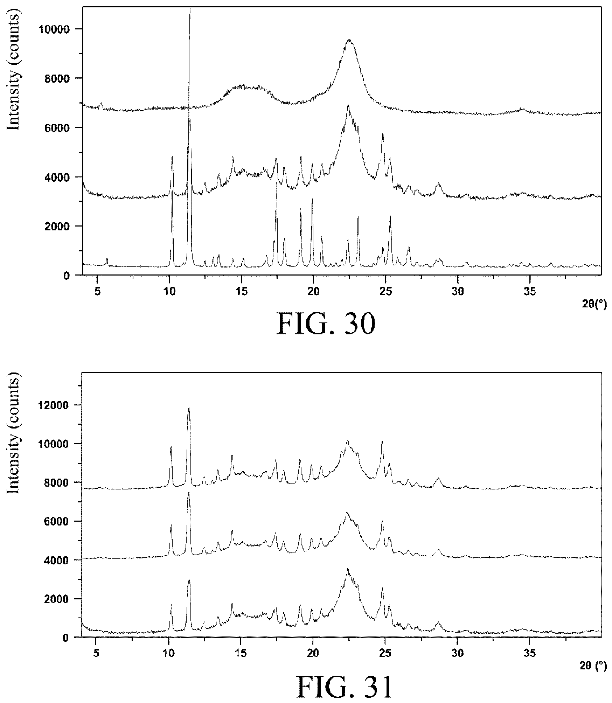

A crystal-form-CSV formulation was prepared by using the formulation formula shown in Table 8 and the formulation process shown in Table 10, and an excipient formula is shown in Table 9. A mixture of excipients and the sample before and after the formulation formulas were tested by XRPD, and the result is shown in FIG. 30. The result shows that crystal form CSV is not changed after the formulation formula processes.

### Example 27 Stability of Crystal-Form-CSV Formulation

A sample of a crystal-form-CSV formulation was packaged under corresponding conditions and stored at 25°C/60% RH, and 40°C/75% RH, and the purity and crystal form were determined by HPLC and XRPD. The result is shown in Table 17, and the XRPD patterns before and after storage are shown in FIG. 31. The result shows that the crystal-form-CSV formulation sample is stable for at least 1 month at 25°C/60% RH and 40°C/75% RH with little change in purity.

**Table 17**

| Storage Condition | Packaging Condition | Storage Time | Crystal Form | Purity (%) |
|---|---|---|---|---|
| Initial | - | - | Crystal Form CSV | 99.99 |
| 25°C/60% RH | Sealed | 1 Month | Crystal Form CSV | 99.99 |
| 40°C/75% RH | Sealed | 1 Month | Crystal Form CSV | 99.99 |

The above embodiments merely illustrate the technical concept and feature of the present invention, and are intended to enable those skilled in the art to understand and implement the present invention, without limitation of the protection scope of the present invention. Any equivalent variations or modifications made according to the spirit of the present invention shall be covered by the protection scope of the present invention.

## Claims

1. A crystal form CSI of Compound I, **characterized in that** an X-ray powder diffraction pattern thereof comprises characteristic peaks at 2θ values of 11.9°±0.2°, 5.9°±0.2°, and 9.5°±0.2° using Cu-Kα radiation,

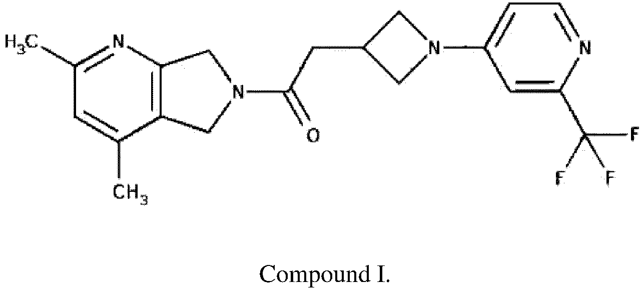

2. The crystal form CSI of Compound I according to claim 1, wherein the X-ray powder diffraction pattern comprises a characteristic peak at a 2θ value of at least one of 10.7°±0.2°, 14.1°±0.2°, and 22.2°±0.2° using Cu-Kα radiation.

3. The crystal form CSI of Compound I according to claim 1, wherein the X-ray powder diffraction pattern comprises a characteristic peak at a 2θ value of at least one of 10.4°±0.2°, 16.3°±0.2°, and 19.1°±0.2° using Cu-Kα radiation.

4. The crystal form CSI of Compound I according to claim 2, wherein X-ray powder diffraction pattern comprises a characteristic peak at a 2θ value of at least one of 10.4°±0.2°, 16.3°±0.2°, and 19.1°±0.2° using Cu-Kα radiation.

5. The crystal form CSI of Compound I according to claim 1, wherein the X-ray powder diffraction pattern is substantially as shown in FIG. 1 using Cu-Kα radiation.

6. A crystal form CSII of Compound I, **characterized in that** an X-ray powder diffraction pattern thereof comprises characteristic peaks at 2θ values of 12.8°±0.2°, 19.8°±0.2°, 24.4°±0.2° using Cu-Kα radiation,

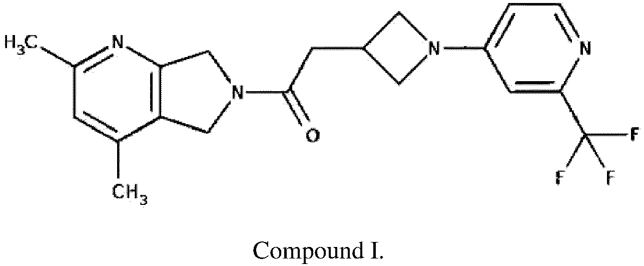

7. The crystal form CSII of Compound I according to claim 6, wherein the X-ray powder diffraction pattern comprises a characteristic peak at a 2θ value of at least one of 21.5°±0.2°, 22.5°±0.2°, and 23.3°±0.2° using Cu-Kα radiation.

8. The crystal form CSII of Compound I according to claim 6, wherein the X-ray powder diffraction pattern comprises a characteristic peak at a 2θ value of at least one of 19.2°±0.2°, 20.8°±0.2°, and 25.8°±0.2° using Cu-Kα radiation.

9. The crystal form CSII of Compound I according to claim 7, wherein the X-ray powder diffraction pattern comprises a characteristic peak at a 2θ value of at least one of 19.2°±0.2°, 20.8°±0.2°, and 25.8°±0.2° using Cu-Kα radiation.

10. The crystal form CSII of Compound I according to claim 6, wherein the X-ray powder diffraction pattern is substantially as shown in FIG. 2 using Cu-Kα radiation.

11. A crystal form CSIII of Compound I, **characterized in that** an X-ray powder diffraction pattern thereof comprises characteristic peaks at 2θ values of 12.5°±0.2°, 6.2°±0.2°, 18.8°±0.2°, and 17.5°±0.2° using Cu-Kα radiation,

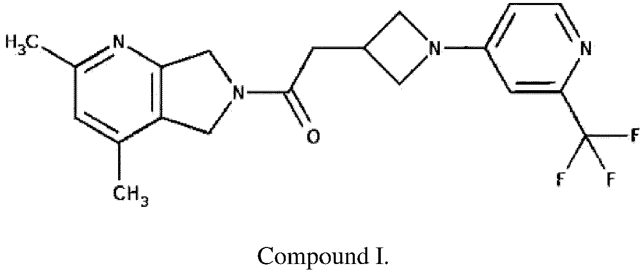

12. The crystal form CSIII of Compound I according to claim 11, wherein the X-ray powder diffraction pattern comprises a characteristic peak at a 2θ value of at least one of 18.1°±0.2°, and 21.7°±0.2° using Cu-Kα radiation.

13. The crystal form CSIII of Compound I according to claim 11, wherein the X-ray powder diffraction pattern is substantially as shown in FIG. 5 using Cu-Kα radiation.

14. A crystal form CSIV of Compound I, **characterized in that** an X-ray powder diffraction pattern thereof comprises characteristic peaks at 2θ values of 13.0°±0.2°, 16.4°±0.2°, 23.1°±0.2°, and 22.0°±0.2° using Cu-Kα radiation,

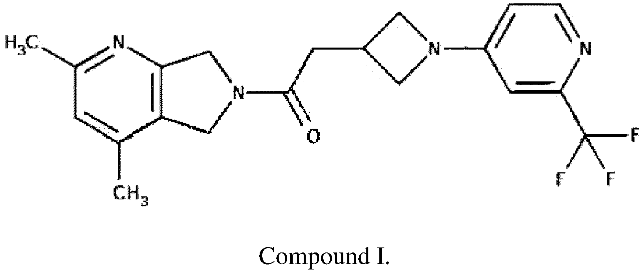

15. The crystal form CSIV of Compound I according to claim 14, wherein the X-ray powder diffraction pattern comprises a characteristic peak at a 2θ value of at least one of 24.0°±0.2°, 14.2°±0.2°, and 17.2°±0.2° using Cu-Kα radiation.

16. The crystal form CSIV of Compound I according to claim 14, wherein the X-ray powder diffraction pattern comprises a characteristic peak at a 2θ value of at least one of 18.1°±0.2°, 20.8°±0.2°, and 27.2°±0.2° using Cu-Kα radiation.

17. The crystal form CSIV of Compound I according to claim 15, wherein the X-ray powder diffraction pattern comprises a characteristic peak at a 2θ value of at least one of 18.1°±0.2°, 20.8°±0.2°, and 27.2°±0.2° using Cu-Kα radiation.

18. The crystal form CSIV of Compound I according to claim 14, wherein the X-ray powder diffraction pattern is substantially as shown in FIG. 7 using Cu-Kα radiation.

19. A crystal form CSV of Compound I, **characterized in that** an X-ray powder diffraction pattern thereof comprises characteristic peaks at 2θ values of 10.3°±0.2°, 19.2°±0.2°, 13.5°±0.2°, and 14.5°±0.2°using Cu-Kα radiation,

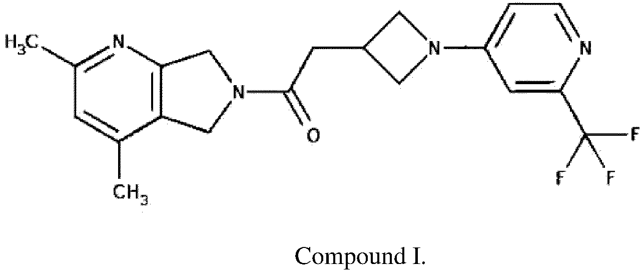

20. The crystal form CSV of Compound I according to claim 19, wherein the X-ray powder diffraction pattern comprises a characteristic peak at a 2θ value of at least one of 18.1°±0.2°, and 20.0°±0.2° using Cu-Kα radiation.

21. The crystal form CSV of Compound I according to claim 19, wherein the X-ray powder diffraction pattern comprises a characteristic peak at a 2θ value of at least one of 20.6°±0.2°, and 12.5°±0.2° using Cu-Kα radiation.

22. The crystal form CSV of Compound I according to claim 20, wherein the X-ray powder diffraction pattern comprises a characteristic peak at a 2θ value of at least one of 20.6°±0.2°, and 12.5°±0.2° using Cu-Kα radiation.

23. The crystal form CSV of Compound I according to claim 19, wherein the X-ray powder diffraction pattern is substantially as shown in FIG. 9 using Cu-Kα radiation.

24. A crystal form of Compound I, **characterized in that** the crystal form is an anhydrate.

25. A pharmaceutical composition comprising a therapeutically effective amount of the crystal form of Compound I according to claims 1, 6, 11, 14, and/or 19, and a pharmaceutically acceptable excipient.

26. Use of the crystal form of Compound I according to claims 1, 6, 11, 14, and/or 19 in preparation of an M4 receptor modulator drug.

27. Use of the crystal form of Compound I according to claims 1, 6, 11, 14, and/or 19 in preparation of a drug for the treatment of schizophrenia.
